# EUROPEAN PATENT APPLICATION

(11) **EP 4 678 191 A1**
(43) Date of publication of application: **14.01.2026**
(21) Application number: 24766532.6
(22) Date of filing: 08.03.2024
(51) Int. Cl.: A61K 47/68, A61K 47/65, A61K 38/08, A61K 38/07, A61P 35/00, A61P 35/02

(54) **USE OF ANTI-CD20 ANTIBODY-DRUG CONJUGATE IN PREPARATION OF DRUG FOR TREATING NON-HODGKIN LYMPHOMA**

(30) Priority: 09.03.2023 CN 202310223225
(71) Applicant: Zhejiang Teruisi Pharmaceutical Inc., Huzhou, Zhejiang 313000 (CN)
(72) Inventor: HUANG, Kai, Huzhou, Zhejiang 313000 (CN); ZHANG, Jian, Huzhou, Zhejiang 313000 (CN); WANG, Chen, Huzhou, Zhejiang 313000 (CN); SHI, Mingbiao, Huzhou, Zhejiang 313000 (CN)
(74) Representative: Haseltine Lake Kempner LLP
(86) International application number: PCT/CN2024/080785
(87) International publication number: WO 2024/183818

(57) **Abstract**

The present disclosure discloses a use of anti-CD20 ADC in the preparation of a drug for treating NHL, and the drug has excellent clinical efficacy and safety in treating a R/R NHL patient after receiving at least two standard treatments.

## Description

### CROSS-REFERENCE TO RELATED APPLICATIONS

This application claims the benefit of priority to the China Invention Patent Application No. CN202310223225.2 filed on March 09, 2023, the entire contents of the aforementioned application are incorporated herein by reference.

### TECHNICAL FIELD

The present disclosure relates to the field of pharmaceutical technology, and particularly relates to the use of anti-CD20 antibody drug conjugate (ADC) in the preparation of a drug for treating a patient with Non-Hodgkin Lymphoma (NHL).

### BACKGROUND

Non-Hodgkin Lymphoma (NHL) is the seventh most common type of cancer in the world, accounting for ~4% to 5% of new cancer cases and 3% to 4% of cancer-related deaths, affecting ~1.5 million people worldwide. NHL is a general term for a group of malignant proliferative diseases of the lymphatic system, including many types of lymphocytomas, which mainly originate from B lymphocytes (> 85%), and a few originate from T cells and natural killer cells. B-cell NHL subtypes include diffuse large B-cell lymphoma (DLBCL), mantle cell lymphoma (MCL), follicular lymphoma (FL), marginal zone lymphoma (MZL), chronic lymphocytic leukemia/small lymphocytic lymphoma (CLL/SLL), and Burkitt lymphoma (BL). DLBCL is the most common type of NHL. It is usually classified clinically using Han's classification, which is divided into germinal center B cell-like (GCB) type and non-germinal center B cell-like (non-GCB) type. Currently, the first-line recommended treatment regimen includes R-CHOP (Rituximab, Cyclophosphamide, Doxorubicin, Vincristine, and Prednisone combined). For relapsed or refractory (R/R) DLBCL, a second-line regimen of chemotherapy with other drugs that are not cross-resistant to CHOP ± Rituximab or an individualized regimen may be selected. If the patient is eligible for transplantation and achieves complete response (CR) or partial response (PR), hematopoietic stem cell transplantation (HSCT) ± local radiotherapy (RT, 30 - 40 Gy) will be performed after chemotherapy, or enroll in a clinical trial; if the patient is not eligible for transplantation or the disease status remains stable or progressive after treatment, enter a clinical trial or receive optimal supportive care.

However, 10% to 15% of patients treated with the R-CHOP regimen have primary refractory disease (i.e., incomplete response or relapse within 6 months of treatment), and another 20% to 25% have progression of disease within 24 months (POD24) after initial response, these patients have poor outcomes. Patients with long-term recurrence (> 2 years after treatment) have a better outcome, but may experience recurrence of indolent lymphoma. For R/R patients who are sensitive to chemotherapy, high-dose chemotherapy and autologous hematopoietic stem cell transplantation (ASCT) may achieve disease cure. However, due to advanced age and comorbidities, only half of relapsed and refractory patients are suitable for ASCT.

Research by Crump M et al. shows that ~73% of relapsed/refractory DLBCL cannot be relieved after second-line or later-line treatment. The SCHOLAR-1 study included 603 patients to assess the prognosis of patients with relapsed/refractory DLBCL. The results showed an objective response rate (ORR) of 26% and a median OS of only 6.3 months, which was not satisfactory (Crump M, Neelapu SS, Farooq U, et al., Outcomes in refractory diffuse large B-cell lymphoma: results from the international SCHOLAR-1 study, Blood, 2017; 130(16):1800-180).

A multicenter real-world (REAL-TREND) study conducted in China also showed similar results to the study by Crump M et al., which included 2,778 patients with DLBCL over 5 years. The cumulative proportion of patients with refractory DLBCL over 5 years was estimated to be 20%, while these patients with refractory DLBCL had very low response rates and OS, with an ORR of only 30%. The complete response rate (CRR) was 9%, the median OS was only 5.9 months, and the 2-year OS rate was only 16% (Wang et al., Outcomes in refractory diffuse large B-cell lymphoma: results from a multicenter real-world study in China, Cancer Communications, 2021;41:229-239).

Therefore, there is still a need to find more effective drugs and treatments for R/R DLBCL.

### SUMMARY

The object of the present disclosure is to provide a use of anti-CD20 antibody drug conjugate (ADC) in the preparation of a drug for treating Non-Hodgkin Lymphoma (NHL). The anti-CD20 ADC has good clinical efficacy and safety in treating relapsed or refractory (R/R) NHL after receiving at least two standard treatments, especially diffuse large B-cell lymphoma (DLBCL).

### A first aspect of the present disclosure provides a use of anti-CD20 ADC in the preparation of a drug for treating an NHL patient.

The anti-CD20 ADC has the following structure:

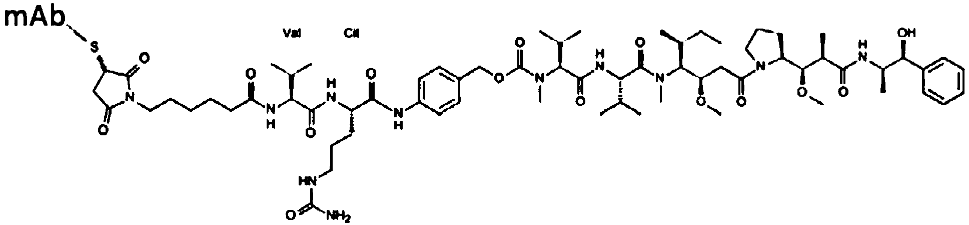

Wherein mAb is Rituximab or a biosimilar thereof, and the drug-antibody ratio (DAR) is 1 to 8. Preferably, DAR is 4.2 ± 1; further preferably, DAR is 4.2 ± 0.5; and still more preferably, DAR is 4.2 ± 0.3.

According to specific embodiments of the present disclosure, the anti-CD20 ADC can be "TRS005", and a preparation method thereof is shown in the Patent Application Publication CN108452319A, the entire contents of which are incorporated in the present disclosure.

The drug for treating NHL patients described in the present disclosure can also be prepared from a pharmaceutical preparation containing the anti-CD20 ADC and a carrier or excipient, wherein the pharmaceutical preparation is a liquid preparation or a freeze-dried preparation.

The carrier or excipient is selected from the group consisting of a pH buffer, an osmotic pressure regulator, a lyophilized powder excipient, a protein protecting agent, a solubilizer, and water for injection (WFI).

The pH buffer includes histidine hydrochloride buffer, acetic acid buffer, phosphate buffer, citric acid buffer, or Tris buffer; preferably, the pH buffer is histidine hydrochloride buffer.

The protein protecting agent includes trehalose, sucrose, lactose, or glucose; preferably, the protein protecting agent is sucrose.

The osmotic pressure regulator includes mannitol or sodium chloride; preferably, the osmotic pressure regulator is mannitol.

The solubilizer includes polysorbate 80, polysorbate 20, or poloxamer 188; preferably, the solubilizer is polysorbate 80.

Further, the liquid or freeze-dried preparation includes the above-mentioned anti-CD20 ADC, histidine hydrochloride, trehalose, mannitol, and polysorbate 80.

The NHL patients described in the present disclosure are R/R NHL patients after receiving at least two standard treatments, including but not limited to FL patients, MZL patients, DLBCL patients, MCL patients, and SLL/CLL patients.

In some embodiments, the NHL patients described in the present disclosure are R/R DLBCL patients after receiving at least two standard treatments;
Further, the DLBCL patients are GCB type DLBCL patients and/or non-GCB type DLBCL patients;
Further, the DLBCL patients are non-GCB type DLBCL patients.

In some embodiments, the DLBCL patients are DLBCL patients with a baseline lactic dehydrogenase (LDH) of ≥ 250 and/or < 250;
Further, the DLBCL patients are DLBCL patients with a baseline LDH of ≥ 250.

In some embodiments, the DLBCL patients are DLBCL patients with a baseline age of ≥ 60 and/or < 60;
Further, the DLBCL patients are DLBCL patients with a baseline age of ≥ 60.

In some embodiments, the DLBCL patients are DLBCL patients with a baseline clinical phase of ≥ III and/or ≤ II;
Further, the DLBCL patients are DLBCL patients with a baseline clinical phase of ≥ III.

### A second aspect of the present disclosure provides a method for treating an NHL patient.

The method comprises administering an effective dose of an anti-CD20 ADC to a patient, and the anti-CD20 ADC has the following structure:

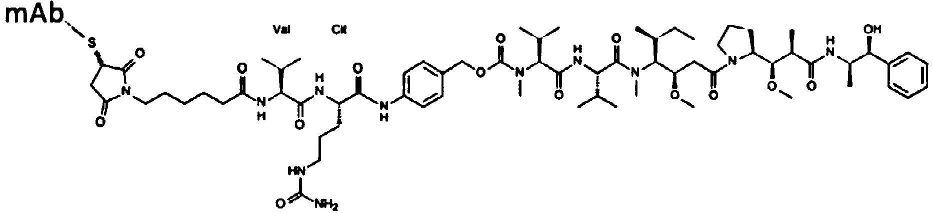

Wherein mAb is Rituximab or a biosimilar thereof, and the drug-antibody ratio (DAR) is 1 to 8. Preferably, DAR is 4.2 ± 1; further preferably, DAR is 4.2 ± 0.5; and still more preferably, DAR is 4.2 ± 0.3.

According to specific embodiments of the present disclosure, the anti-CD20 ADC can be "TRS005", and a preparation method thereof is shown in the Patent Application Publication CN108452319A, the entire contents of which are incorporated in the present disclosure.

When administering the anti-CD20 ADC to the NHL patients described in the present disclosure, it can also be administered in the form of a pharmaceutical preparation containing the anti-CD20 ADC and a carrier or excipient, and the pharmaceutical preparation is a liquid preparation or a freeze-dried preparation.

The carrier or excipient is selected from the group consisting of a pH buffer, an osmotic pressure regulator, a lyophilized powder excipient, a protein protecting agent, a solubilizer, and water for injection (WFI).

The pH buffer includes histidine hydrochloride buffer, acetic acid buffer, phosphate buffer, citric acid buffer, or Tris buffer; preferably, the pH buffer is histidine hydrochloride buffer.

The protein protecting agent includes trehalose, sucrose, lactose, or glucose; preferably, the protein protecting agent is sucrose.

The osmotic pressure regulator includes mannitol or sodium chloride; preferably, the osmotic pressure regulator is mannitol.

The solubilizer includes polysorbate 80, polysorbate 20, or poloxamer 188; preferably, the solubilizer is polysorbate 80.

Further, the liquid or freeze-dried preparation includes the above-mentioned anti-CD20 ADC, histidine hydrochloride, trehalose, mannitol, and polysorbate 80.

In some embodiments, the dosage of the anti-CD20 ADC is 0.05 mg/kg to 2.7 mg/kg.

Preferably, the dosage of the anti-CD20 ADC is 0.1 mg/kg to 2.3 mg/kg.

Preferably, the dosage of the anti-CD20 ADC is 0.1 mg/kg, 0.5 mg/kg, 1 mg/kg, 1.3 mg/kg, 1.5 mg/kg, 1.8 mg/kg, 2.1 mg/kg, and 2.3 mg/kg.

More preferably, the dosage of the anti-CD20 ADC is 0.5 mg/kg, 1 mg/kg, 1.5 mg/kg, 1.8 mg/kg, and 2.1 mg/kg.

Most preferably, the dosage of the anti-CD20 ADC is 1.8 mg/kg.

The NHL patients are R/R NHL patients after receiving at least two standard treatments, including FL patients, MZL patients, DLBCL patients, MCL patients, and SLL/CLL patients.

In some embodiments, the NHL patients described in the present disclosure are R/R DLBCL patients after receiving at least two standard treatments;

Further, the DLBCL patients are GCB type DLBCL patients and/or non-GCB type DLBCL patients;

Further, the DLBCL patients are non-GCB type DLBCL patients.

Further, the DLBCL patients are non-GCB type DLBCL patients, and the dosage of the anti-CD20 ADC is 1.5 mg/kg or 1.8 mg/kg.

In some embodiments, the DLBCL patients are DLBCL patients with a baseline lactic dehydrogenase (LDH) of ≥ 250 and/or < 250;

Further, the DLBCL patients are DLBCL patients with a baseline LDH of ≥ 250.

Further, the DLBCL patients are DLBCL patients with a baseline LDH of ≥ 250, and the dosage of the anti-CD20 ADC is 1.8 mg/kg.

In some embodiments, the DLBCL patients are DLBCL patients with a baseline age of ≥ 60 and/or < 60;

Further, the DLBCL patients are DLBCL patients with a baseline age of ≥ 60.

Further, the DLBCL patients are DLBCL patients with a baseline age of ≥ 60, and the dosage of the anti-CD20 ADC is 1.8 mg/kg.

In some embodiments, the DLBCL patients are DLBCL patients with a baseline clinical phase of ≥ III and/or ≤ II;

Further, the DLBCL patients are DLBCL patients with a baseline clinical phase of ≥ III.

Further, the DLBCL patients are DLBCL patients with a baseline clinical phase of ≥ III, and the dosage of the anti-CD20 ADC is 1.8 mg/kg.

In some embodiments, the anti-CD20 ADC can be administered once or multiple times.

Preferably, the anti-CD20 ADC is administered multiple times, and the administration frequency can be QW, Q2W, Q3W, or Q4W.

More preferably, the administration frequency is Q3W. Studies have shown that the pharmacokinetic (PK) characteristics of the anti-CD20 ADC of the present disclosure are similar after a single dose and four cycles of administration, and it is almost completely metabolized in the blood after 21 days, supporting the preferred administration frequency of Q3W.

In some embodiments, the interval between each administration is one treatment cycle, and the administration time can be 1 to 20 treatment cycles; preferably, 6 treatment cycles.

In some embodiments, the anti-CD20 ADC is administered Q3W and continues to be administered until recovered, progressive disease (PD), or death.

In some embodiments, the administration method can be intramuscular, intravenous, intra-arterial, intraperitoneal, or subcutaneous administration; preferably, intravenous administration.

It is to be understood that one, some, or all of the properties of the various embodiments described herein may be combined to form other embodiments of the present disclosure. Specific embodiments of the present disclosure are further described in details as follows.

### DESCRIPTION OF EMBODIMENTS

The present disclosure is described below with reference to specific embodiments. It will be appreciated by those skilled in the art that these embodiments are for illustration of the disclosure only and are not intended to limit the scope of the present disclosure in any way.

The experimental methods in the following embodiments are conventional methods unless otherwise specified. The raw materials, reagent materials, and the like used in the embodiments described below are commercially available products unless otherwise specified.

### Definition:

"Rituximab" refers to a monoclonal antibody drug targeting CD20 developed by Roche and approved for marketing by the National Medical Products Administration (NMPA) under the trade name of "MabThera". Rituximab is the generic name, and only the innovator drug can be called Rituximab.

"Rituximab biosimilar" refers to the monoclonal antibody drug developed by other companies in addition to the innovator drug of Rituximab, with identical amino acid sequences and comparable physical and chemical properties, efficacy, pharmacokinetics, and safety to the innovator drug.

"Complete Response (CR)" means that the target lesion disappears completely, or all target nodules must be reduced to normal size (short axis < 10 mm), and last for 4 weeks or more.

"Partial Response (PR)" means that the sum of the diameters of all measurable target lesions (long diameter for target lesions and short diameter for target nodules) is reduced by ≥ 50% compared with baseline (in hematological tumors), which lasts for 4 weeks or more.

"Progressive Disease (PD)" means that the minimum value of the sum of all measured diameters of target lesions during the entire tumor treatment process is used as a reference value. The sum of the diameters of target lesions exceeds 20% or more of the reference value, and the absolute value increases by 5 mm or more, or one or more new lesions appear, and CR and PR are not achieved before the size or number of lesions increase.

"Stable Disease (SD)" means that changes in the volume and number of lesions between PR and PD.

"Overall Survival (OS)" refers to the time from randomization (receiving group treatment) to the death or loss to follow-up of the subject for any reason.

"Progression-Free Survival (PFS)" refers to the time from randomization to the first tumor recurrence/metastasis, or death of the subject due to any other reason.

"Median Progression-Free Survival (mPFS)" refers to the PFS that can be achieved in 50% of evaluable patients.

"Objective Response Rate (ORR)" refers to the sum of the CRR and the partial response rate (PRR), that is, the proportion of patients who have achieved CR and PR to all evaluable patients.

"Disease control rate (DCR)" refers to the sum of CRR, PRR, and SD rate, that is, the proportion of patients who have achieved CR, PR, and SD to all evaluable patients.

"Refractory" means that PR is not achieved in two treatment cycles, or CR is not achieved in 4 treatment cycles.

"Effective dose" refers to the amount that effectively achieves the desired treatment result at the required dose and time period. The effective dose may vary depending on factors such as the individual's disease state, age, gender, and weight, and the ability of the therapeutic agent or combination of therapeutic agents to induce a desired response in the individual.

"Standard treatment": In the present disclosure, it refers not only to the standard treatment regimen as determined in the conventional clinical sense, but also includes clinically recommended treatment plans, clinical trial treatment plans, and other treatment plans.

Although the present disclosure has been generally described, embodiments of the present disclosure will be further disclosed in the following embodiments, which should not be construed as limiting the scope of the claims.

### Embodiment 1: Preparation of Anti-CD20 ADC (TRS005)

The preparation method of TRS005 is shown in the Patent Application Publication CN108452319A, the entire contents of which are incorporated in the present disclosure.

The structure of TRS005 is as follows:

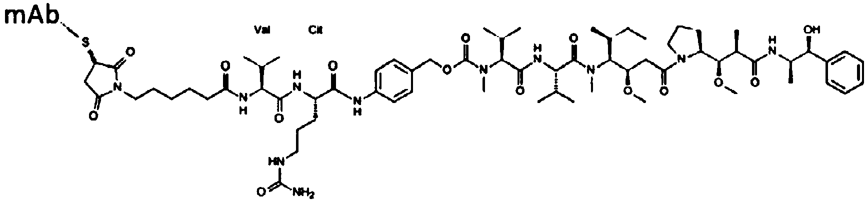

Wherein mAb is Rituximab or its biosimilar, and the drug antibody ratio (DAR) is 4.2 ± 0.5.

### Embodiment 2: Study to Evaluate the Safety, Tolerability, Pharmacokinetics, and Effectiveness of TRS005 in Treatment of R/R NHL

The clinical efficacy data of TRS005 are from A Multicenter, Single-arm, Dose-escalating Study to Evaluate the Safety, Tolerability, Pharmacokinetics and Effectiveness of TRS005 in Patients with Relapsed or Refractory CD20-positive B-NHL. The study enrolled 128 subjects from August 24, 2020 to October 20, 2023, and efficacy analysis was performed on 96 subjects (data updated to October 20, 2023).

### (1) Baseline Information of Subjects

The baseline information of the 128 enrolled subjects is shown in Table 1.

**Table 1 Baseline Information of R/R NHL Subjects**

| **Baseline Characteristics** | | **Subjects Enrolled (N = 128, %)** |
|---|---|---|
| **Age (Median)** | | 60 |
| | Range | 25-83 |
| | ≥ 60 | 67 (52.3%) |
| | < 60 | 61 (47.7%) |

| **Gender** | | |
|---|---|---|
| | Male | 76(59.4%) |
| | Female | 52(40.6%) |

| **Histological Subtype** | | |
|---|---|---|
| | FL | 19 (14.8%) |
| | DLBCL | 69 (53.9%) |
| | MCL | 11(8.6%) |
| | MZL | 4 (3.1%) |
| | SLL/ CLL | 5 (3.9%) |
| | Others | 20 (15.6%) |

| **ECOG Score** | | |
|---|---|---|
| | 0 | 48 (37.5%) |
| | 1 | 80 (62.5%) |

| **Lugano Clinical Phase** | | |
|---|---|---|
| | I | 3 (2.3%) |
| | II | 13 (10.2%) |
| | III | 38 (29.7%) |
| | IV | 67 (52.3%) |
| | Others | 7 (5.5%) |

| **Prior Line of Treatment** | | |
|---|---|---|
| | 2 to 3 lines | 106 (82.8%) |
| | ≥ 4 lines | 21 (17.2%) |

### (2) Administration Regimen

The doses administered in the dose escalation stage of this study are 0.1 mg/kg, 0.5 mg/kg, 1.0 mg/kg, 1.5 mg/kg, 1.8 mg/kg, and 2.1 mg/kg, and in the dose expansion stage is 1.5 mg/kg and 1.8 mg/kg. TRS005 will be administered intravenously on Day 1 of each cycle (21 days as a cycle, a total of 6 cycles). Patients with clinical benefits will continue to receive medication until recovered, PD, or death.

### (3) Efficacy Data

Of the 128 subjects enrolled in Phase I (dose group: 0.1 to 2.1 mg/kg), 96 patients underwent analysis of efficacy after treatment. The overall efficacy of the study was assessed as CR in 17 subjects and PR in 26 subjects, with an ORR of 44.8% (43/96). Overall, TRS005 has achieved excellent clinical efficacy for R/R NHL patients after receiving at least two standard treatments.

By histological subtype, the ORR of DLBCL was 48.2% (27/56, including 12 CRs and 15 PRs), the ORR of MCL was 55.6% (5/9, including 2 CRs and 3 PRs), and the ORR of FL was 29.4% (5/17, including 2 CRs and 3 PRs). Based on the results, TRS005 has demonstrated excellent efficacy in the treatment of R/R DLBCL and MCL after second-line or later therapies. This superior performance of TRS005 in DLBCL and MCL was unexpected. The data are shown in Table 2.

**Table 2 Efficacy Data of R/R NHL Subjects with Different Pathological Subtypes**

| **Pathological Subtype** | **Total Number of Subjects** | **Dose Group** | **Number of Subjects** | **CR** | **PR** | **SD** | **PD** | **ORR%** | **CR%** | **PR%** | **DCR%** |
|---|---|---|---|---|---|---|---|---|---|---|---|
| | | **mg/kg** | | | | | | | | | |
| **DLBCL** | 56 | 0.5 | 3 | 1 | 0 | 0 | 2 | 48.2% | 21.4% | 26.8% | 76.8% |
| | | 1 | 2 | 0 | 0 | 1 | 1 | | | | |
| | | 1.5 | 21 | 3 | 6 | 6 | 6 | | | | |
| | | 1.8 | 30 | 8 | 9 | 9 | 4 | | | | |
| | | 0.5 | 4 | 0 | 2 | 2 | 0 | | | | |
| | | 1 | 3 | 0 | 1 | 1 | 1 | | | | |
| **FL** | 17 | 1.5 | 3 | 0 | 0 | 1 | 2 | 29.4% | 11.8% | 17.6% | 70.6% |
| | | 1.8 | 7 | 2 | 0 | 3 | 2 | | | | |
| | | 1.5 | 2 | 0 | 2 | 0 | 0 | | | | |
| **MCL** | 9 | 1.8 | 6 | 2 | 1 | 2 | 1 | 55.6% | 22.2% | 33.3% | 88.9% |
| | | 2.1 | 1 | 0 | 0 | 1 | 0 | | | | |
| **MZL** | 1 | 1.8 | 1 | 0 | 0 | 1 | 0 | 0.0% | 0.0% | 0.0% | 100.0% |
| **SLL/CLL** | 2 | 0.1 | 1 | 0 | 0 | 1 | 0 | 0.0% | 0.0% | 0.0% | 50.0% |
| | | 1.5 | 1 | 0 | 0 | 0 | 1 | | | | |
| | | 1 | 1 | 0 | 1 | 0 | 0 | | | | |
| **Others** | 11 | 1.5 | 2 | 0 | 0 | 1 | 1 | 45.5% | 9.1% | 36.4% | 81.8% |
| | | 1.8 | 8 | 1 | 3 | 3 | 1 | | | | |

| | | | | | | | | | | | |
|---|---|---|---|---|---|---|---|---|---|---|---|
| Abbreviations: CR: Complete response; PR: Partial response; SD: Stable disease; PD: Progressive disease; ORR: Objective response rate; DCR: Disease control rate. | | | | | | | | | | | |

From the perspective of each dose group, the ORR was 42.9% in the 0.5 mg/kg dose group, 41.4% in the 1.5 mg/kg dose group, and 50.0% in the 1.8 mg/kg dose group. It can be seen that the efficacy can also be observed in the low-dose group, but the efficacy signal is the best in the 1.8 mg/kg dose group. The data are shown in Table 3.

**Table 3 Efficacy Data of R/R NHL Subjects in Different Dose Groups**

| **Best Overall Response (BOR)** | **0.1 mg/kg (N = 1)** | **0.5 mg/kg (N = 7)** | **1.0 mg/kg (N = 6)** | **1.5 mg/kg (N = 29)** | **1.8 mg/kg (N = 52)** | **2.1 mg/kg (N = 1)** | **Total (N = 96)** |
|---|---|---|---|---|---|---|---|
| CR-n, (%) | 0 | 1 (14.3) | 0 | 3 (9.1) | 13 (25.0) | 0 | 17 (17.7) |
| PR-n, (%) | 0 | 2 (28.6) | 2 (33.3) | 9 (27.3) | 13 (25.0) | 0 | 26 (27.1) |
| SD-n, (%) | 1 (100.0) | 2 (28.6) | 2 (33.3) | 8 (24.2) | 18 (34.6) | 1 (100.0) | 32 (33.3) |
| PD-n, (%) | 0 | 2 (28.6) | 2 (33.3) | 9 (27.3) | 8 (15.4) | 0 | 21 (21.9) |
| ORR-% | 0 | 42.9 | 33.3 | 41.4 | 50.0 | 0 | 44.79 |
| DCR-% | 100.0 | 71.4 | 66.7 | 69.0 | 84.6 | 100.0 | 78.1 |

| | | | | | | | |
|---|---|---|---|---|---|---|---|
| Abbreviations: CR: Complete response; PR: Partial response; SD: Stable disease; PD: Progressive disease; ORR: Objective response rate; DCR: Disease control rate. | | | | | | | |

### (4) Safety

All treatment-emergent adverse events (TEAEs) (data as of September 18, 2023): 1535 cases in 93 (83.0%) subjects. Treatment-related adverse events (TRAEs): 1303 cases in 91 (81.3%) subjects. ≥ Grade 3 TEAEs: 204 cases in 68 (60.7%) subjects, of which 186 cases in 64 (57.1%) subjects were determined to be related to the investigational drug.

Adverse reactions with an incidence of ≥ 10% during the study mainly included hematological toxicities (such as white blood cell count decreased, neutrophil count decreased, anaemia, lymphocyte count decreased, and platelet count decreased) and non-hematological toxicities (such as AST increased, ALT increased, pyrexia, asthenia, nausea, vomiting, and hypokalaemia). Among them, the main TRAEs of Grade 3 or higher were hematological toxicities. No adverse reactions of Grade 3 or higher with an incidence of ≥ 10% were observed in relation to cardiac, renal, hepatic, gastrointestinal, dermatological, neurological, or pulmonary toxicities.

In summary, the safety profile of TRS005 in Phase I clinical study is similar to that of the approved control drug Rituximab, and there is no significant difference in the type and frequency of occurrence. The comprehensive evaluation shows that TRS005 is safe and well tolerated.

### Embodiment 3: Efficacy Analysis of TRS005 1.5 mg/kg and 1.8 mg/kg Dose Groups in the Treatment of R/R DLBCL.

### (1) Baseline Information of Subjects

The baseline information of the 50 enrolled subjects is shown in Table 4.

**Table 4 Baseline Information of R/R DLBCL Subjects in 1.5 mg/kg and 1.8 mg/kg Dose Groups**

| **Baseline Characteristics** | **1.5 mg/kg (N = 21)** | **1.8 mg/kg (N = 29)** | **Total (N** = **50)** |
|---|---|---|---|
| **Age - No. (%)** | | | |
| ≥ 60 | 12 (24.0%) | 11 (22.0%) | 23 (46.0%) |
| < 60 | 9 (18.0%) | 18(36.0%) | 27 (54.0%) |

| **Gender - No. (%)** | | | |
|---|---|---|---|
| Male | 12 (24.0%) | 11 (22.0%) | 23 (46.0%) |
| Female | 9 (18.0%) | 18 (36.0%) | 27 (54.0%) |

| **Tumor Cell Origin** - **No. (%) (1 subject did not have baseline molecular typing)** | | | |
|---|---|---|---|
| GCB | 7 (14.3%) | 8 (16.3%) | 15 (30.6%) |
| non-GCB | 14 (28.6%) | 20 (40.8%) | 34 (69.4%) |

| **ECOG Score** - **No. (%)** | | | |
|---|---|---|---|
| 0 | 6 (12.0%) | 13 (26.0%) | 19 (38.0%) |
| 1 | 15 (30.0%) | 16 (32.0%) | 31 (62.0%) |

| **Lugano Phase** - **No. (%) (7 subjects did not have baseline clinical phase)** | | | |
|---|---|---|---|
| ≤II | 3 (7.0%) | 5 (11.6%) | 8 (18.6%) |
| ≥III | 15 (34.9%) | 20 (46.5%) | 35 (81.4%) |

| **Lines of Anti-tumor Treatment - No. (%)** | | | |
|---|---|---|---|
| 2 | 11 (22.0%) | 16 (32.0%) | 26 (54.0%) |
| >2 | 10 (20.0%) | 13 (26.0%) | 23 (46.0%) |

| **Baseline LDH** | | | |
|---|---|---|---|
| LDH ≥ 250 | 12 (24.0%) | 20 (40.0%) | 32 (64.0%) |
| LDH < 250 | 18 (22.0%) | 18 (22.0%) | 18 (36.0%) |

Data statistics are as of October 20, 2023.

### (2) Overall Efficacy Data

The doses administered in the dose expansion stage of this study were 1.5 mg/kg and 1.8 mg/kg. A total of 50 subjects were evaluable for DLBCL in the above-mentioned two dose groups, with an ORR of 42.9% and a DCR of 71.4% in the 1.5 mg/kg dose group, an ORR of 58.6% and a DCR of 89.7% in the 1.8 mg/kg dose group, and an overall ORR of 52.0% and a DCR of 82.0%. The above data showed that TRS005 achieved excellent efficacy in DLBCL subjects and was dose-dependent. Especially in the proposed recommended phase II dose (RP2D) 1.8 mg/kg dose group, the ORR reached 58.6%, which was significantly better than the results of Crump M et al. and Wang et al. (ORR was 26% and 30%, respectively). The data are shown in Table 5.

**Table 5 Efficacy of R/R DLBCL Subjects in 1.5 mg/kg and 1.8 mg/kg Dose Groups**

| **Best Overall Response** | | **1.5mg/kg (N=21),%** | **1.8mg/kg (N=29), %** | **Total (N = 50, %)** |
|---|---|---|---|---|
| **BOR- n (%)** | | | | |
| | CR | 3 (14.3) | 8 (27.6) | 11 (22.0) |
| | PR | 6 (28.6) | 9 (31.0) | 15 (30.0) |
| | SD | 6 (28.6) | 9 (31.0) | 15 (30.0) |
| | PD | 6 (28.6) | 3 (10.3) | 9 (18.0) |
| **ORR** | | 42.9% | 58.6% | 52.0% |
| **DCR** | | 71.4% | 89.7% | 82.0% |

### (3) Stratified Analysis of Efficacy

### a. Baseline Molecular Typing

DLBCL can be divided into GCB subtype and non-GCB subtype according to the most commonly used Han's classification. Patients with non-GCB DLBCL have a worse prognosis and greater demand for clinical medication, requiring better clinical drugs and treatment options.

A total of 49 patients were included in Han's classification analysis. The study results showed that TRS005 achieved excellent efficacy in both GCB and non-GCB subtypes. Unexpectedly, TRS005 had better efficacy for the non-GCB subtype and was dose-dependent. The ORR of the overall non-GCB subtype was 55.9%, and the ORR of the GCB subtype was 40%. Generally, the prognosis of non-GCB is worse than that of GCB. Unexpectedly, the above results showed that TRS005 had better efficacy on non-GCB and correspondingly achieved a better prognosis compared with GCB, which could provide better drugs and treatment regimens for clinical practice to meet the unmet needs in existing clinical practice. The data are shown in Table 6.

**Table 6 Efficacy of R/R DLBCL Subjects with Different Han's Subtypes**

| **Baseline Characteristics** | **Dose Group** | **BOR - n** | | | | | | | | | | | | | |
|---|---|---|---|---|---|---|---|---|---|---|---|---|---|---|---|
| | | **Number of Subjects** | **CR** | **PR** | **SD** | **PD** | **ORR** | **CRR** | **Number of Subjects** | **CR** | **PR** | **SD** | **PD** | **ORR** | **CRR** |
| **GCB** | 1.5 mg/kg | 7 | 0 | 2 | 3 | 2 | 28.6% | 0.0% | 15 | 2 | 4 | 5 | 4 | 40.0% | 13.3% |
| | 1.8 mg/kg | 8 | 2 | 2 | 2 | 2 | 50.0% | 25.0% | | | | | | | |
| **Non-GCB** | 1.5 mg/kg | 14 | 3 | 4 | 3 | 4 | 50.0% | 21.4% | 34 | 9 | 10 | 10 | 5 | 55.9% | 26.5% |
| | 1.8 mg/kg | 20 | 6 | 6 | 7 | 1 | 60.0% | 30.0% | | | | | | | |

### b. Baseline LDH

Higher LDH levels play an important role in tumor metabolism, proliferation, invasion, and metastasis. Usually, the ORR of patients with low baseline LDH levels is higher than that of patients with high baseline LDH levels.

The study results showed that TRS005 achieved excellent efficacy in patients with a baseline LDH of ≥ 250 (high group) and < 250 (low group). Unexpectedly, the efficacy of TRS005 in subjects with high baseline LDH levels was higher than that in subjects with low baseline LDH levels and was dose-dependent. The overall ORR was 56.3% for patients with a baseline LDH of ≥ 250 and 44.4% for patients with a baseline LDH of < 250. Especially in the 1.8 mg/kg dose group, the ORR of subjects in the high baseline LDH level group reached 65%, while the ORR of subjects in the low baseline LDH level group was 44.4%. The above results showed that, unexpectedly, TRS005 had better efficacy in DLBCL subjects with high baseline LDH levels. The data are shown in Table 7.

**Table 7 Efficacy of R/R DLBCL Subjects with Different Baseline LDH Levels**

| **Baseline Characteristics** | **Dose Group** | **BOR - n** | | | | | | | | | | | | | |
|---|---|---|---|---|---|---|---|---|---|---|---|---|---|---|---|
| | | **Number of Subjects** | **CR** | **PR** | **SD** | **PD** | **ORR** | **CRR** | **Number of Subjects** | **CR** | **PR** | **SD** | **PD** | **ORR** | **CRR** |
| **LDH≥250** | 1.5 mg/kg | 12 | 1 | 4 | 2 | 5 | 41.7% | 8.3% | 32 | 6 | 12 | 6 | 8 | 56.3% | 18.8% |
| | 1.8 mg/kg | 20 | 5 | 8 | 4 | 3 | 65.0% | 25.0% | | | | | | | |
| **LDH <250** | 1.5 mg/kg | 9 | 2 | 2 | 4 | 1 | 44.4% | 22.2% | 18 | 5 | 3 | 9 | 1 | 44.4% | 26.3% |
| | 1.8 mg/kg | 9 | 3 | 1 | 5 | 0 | 44.4% | 33.3% | | | | | | | |

### c. Baseline Age (≥ 60 VS < 60)

The study results showed that TRS005 achieved excellent efficacy in subjects aged ≥ 60 and < 60. Unexpectedly, TRS005 had better efficacy in subjects aged ≥ 60 than in subjects aged < 60 and was dose-dependent. The ORR was 60.9% for subjects aged ≥ 60 and 44.4% for subjects aged < 60. Especially in the 1.8 mg/kg dose group, the ORR for subjects aged ≥ 60 was 72.7% and the CRR was 54.6%, while the ORR for subjects aged < 60 was 50.0% and the CRR was 11.1%. The above results showed that, unexpectedly, TRS005 had better efficacy in elderly subjects with DLBCL. The data are shown in Table 8.

**Table 8 Efficacy of R/R DLBCL Subjects at Different Baseline Ages**

| **Baseline Characteristics** | **Dose Group** | **BOR - n** | | | | | | | | | | | | | |
|---|---|---|---|---|---|---|---|---|---|---|---|---|---|---|---|
| | | **Number of Subjects** | **CR** | **PR** | **SD** | **PD** | **ORR** | **CRR** | **Number of Subjects** | **CR** | **PR** | **SD** | **PD** | **ORR** | **CRR** |
| **Age ≥ 60** | 1.5 mg/kg | 12 | 1 | 5 | 2 | 4 | 50.0% | 8.3% | 23 | 7 | 7 | 3 | 6 | 60.9% | 30.4% |
| | 1.8 mg/kg | 11 | 6 | 2 | 1 | 2 | 72.7% | 54.6% | | | | | | | |
| **Age < 60** | 1.5 mg/kg | 9 | 2 | 1 | 4 | 2 | 33.3% | 22.2% | 27 | 4 | 8 | 12 | 3 | 44.4% | 14.8% |
| | 1.8 mg/kg | 18 | 2 | 7 | 8 | 1 | 50.0% | 11.1% | | | | | | | |

### d. Baseline Clinical Phase (≥ III VS ≤ II)

The study results showed that in the 1.8 mg/kg dose group, the ORR was 70% for subjects with clinical phase ≥ III, while the ORR was only 20% for subjects with clinical phase ≤ II. Therefore, unexpectedly, TRS005 at a dose of 1.8 mg/kg had significantly higher efficacy in subjects with high clinical phases than in subjects with low clinical phases. The data are shown in Table 9.

**Table 9 Efficacy of R/R DLBCL Subjects at Baseline Clinical Phases**

| **Baseline Characteristics** | **Dose Group** | **BOR - n** | | | | | | | | | | | | | |
|---|---|---|---|---|---|---|---|---|---|---|---|---|---|---|---|
| | | **Number of Subjects** | **CR** | **PR** | **SD** | **PD** | **ORR** | **CRR** | **Number of Subjects** | **CR** | **PR** | **SD** | **PD** | **ORR** | **CRR** |
| **Clinical Phase ≤ II** | 1.5 mg/kg | 3 | 1 | 2 | 0 | 0 | 100.0% | 33.3% | 8 | 2 | 2 | 3 | 1 | 50.0% | 25% |
| | 1.8 mg/kg | 5 | 1 | 0 | 3 | 1 | 20.0% | 20.0% | | | | | | | |
| **Clinical Phase ≥ III** | 1.5 mg/kg | 15 | 1 | 3 | 5 | 6 | 26.7% | 6.7% | 35 | 7 | 11 | 9 | 8 | 51.4% | 20% |
| | **1.8 mg/kg** | **20** | **6** | **8** | **4** | **2** | **70.0%** | **30.0%** | | | | | | | |

### e. Prior Line of Treatment

The study results showed that there was no significant difference in the overall efficacy of TRS005 for patients with different prior lines of treatment, as shown in Table 10.

**Table 10 Efficacy of R/R DLBCL Subjects with Different Prior Lines of Treatment at Baseline**

| **Baseline Characteristics** | **Dose Group** | **BOR - n** | | | | | | | | | | | | | |
|---|---|---|---|---|---|---|---|---|---|---|---|---|---|---|---|
| | | **Number of Subjects** | **CR** | **PR** | **SD** | **PD** | **ORR** | **CRR** | **Number of Subjects** | **CR** | **PR** | **SD** | **PD** | **ORR** | **CRR** |
| **Prior Line of Treatment = 2** | 1.5 mg/kg | 11 | 0 | 4 | 4 | 3 | 36.4% | 0.0% | 27 | 6 | 8 | 9 | 4 | 51.9% | 22.2% |
| | 1.8 mg/kg | 16 | 6 | 4 | 5 | 1 | 62.5% | 37.5% | | | | | | | |
| **Prior Line of Treatment > 2** | 1.5 mg/kg | 10 | 3 | 2 | 2 | 3 | 50.0% | 30.0% | 23 | 5 | 7 | 6 | 5 | 52.2% | 21.7% |
| | 1.8 mg/kg | 13 | 2 | 5 | 4 | 2 | 53.9% | 15.4% | | | | | | | |

As shown above, TRS005 showed excellent clinical efficacy overall in DLBCL subjects after receiving at least two standard treatments, especially for DLBCL subjects of non-GCB subtypes, with high baseline LDH levels, and who are elderly. Compared with the 1.5 mg/kg dose group, the 1.8 mg/kg dose group had better efficacy.

In conclusion, TRS005 has shown good clinical efficacy and safety in the treatment of NHL, especially DLBCL, after at least two standard treatments, with excellent drug potential.

The above description of embodiments is not intended to limit the present disclosure. Those skilled in the art may make various changes or modifications according to the present disclosure, and such changes or modifications are intended to fall within the scope of the appended claims if they do not depart from the spirit of the disclosure.

## Claims

1. A use of anti-CD20 antibody drug conjugate (ADC) in the preparation of a drug for treating a Non-Hodgkin Lymphoma (NHL) patient, wherein the anti-CD20 ADC has the following structure: 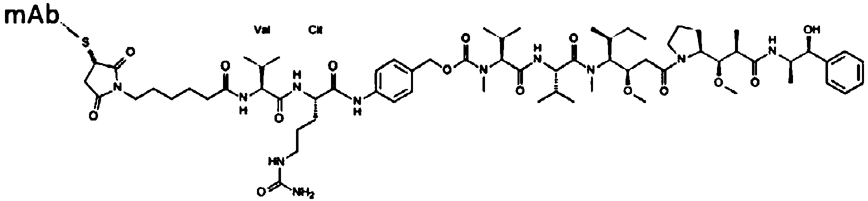 wherein mAb is Rituximab or a biosimilar thereof, and the drug-antibody ratio (DAR) is 1 to 8.

2. The use according to claim 1, wherein the drug antibody ratio (DAR) is 4.2 ± 1; preferably, 4.2 ± 0.5.

3. The use according to claim 1, wherein the DAR is 4.2 ± 0.3.

4. The use according to any one of claims 1 to 3, wherein the NHL patient is a relapsed or refractory (R/R) NHL patient after receiving at least two standard treatments, including follicular lymphoma (FL) patient, marginal zone lymphoma (MZL) patient, diffuse large B-cell lymphoma (DLBCL) patient, mantle cell lymphoma (MCL) patient, and small lymphocytic lymphoma/chronic lymphocytic leukemia (SLL/CLL) patient.

5. The use according to claim 4, wherein the NHL patient is a R/R DLBCL patient after receiving at least two standard treatments.

6. The use according to claim 5, wherein the DLBCL patient is a germinal center B cell-like (GCB) type DLBCL patient and/or a non-germinal center B cell-like (non-GCB) type DLBCL patient.

7. The use according to claim 6, wherein the DLBCL patient is a non-GCB type DLBCL patient.

8. The use according to claim 5, wherein the DLBCL patient is a DLBCL patient with a baseline lactic dehydrogenase (LDH) of ≥ 250 and/or < 250.

9. The use according to claim 8, wherein the DLBCL patient is a DLBCL patient with a baseline LDH of ≥ 250.

10. The use according to claim 5, wherein the DLBCL patient is a DLBCL patient with a baseline age of ≥ 60 and/or < 60.

11. The use according to claim 10, wherein the DLBCL patient is a DLBCL patient with a baseline age of ≥ 60.

12. The use according to claim 5, wherein the DLBCL patient is a DLBCL patient with a baseline clinical phase of ≥ III and/or ≤ II.

13. The use according to claim 12, wherein the DLBCL patient is a DLBCL patient with a baseline clinical phase of ≥ III.

14. A method for treating an NHL patient, wherein the method comprises administering an effective dose of an anti-CD20 ADC to a patient, and the anti-CD20 ADC has the following structure: 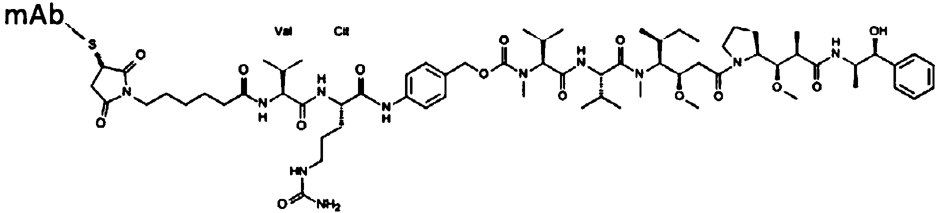 Wherein mAb is Rituximab or a biosimilar thereof, and the DAR is 1 to 8; preferably, DAR is 4.2 ± 1; further preferably, DAR is 4.2 ± 0.5; and still more preferably, DAR is 4.2 ± 0.3.

15. The method according to claim 14, wherein the dosage of the anti-CD20 ADC is 0.05 mg/kg to 2.7 mg/kg; preferably, the dosage of the anti-CD20 ADC is 0.1 mg/kg to 2.3 mg/kg; preferably, the dosage of the anti-CD20 ADC is 0.1 mg/kg, 0.5 mg/kg, 1 mg/kg, 1.3 mg/kg, 1.5 mg/kg, 1.8 mg/kg, 2.1 mg/kg, and 2.3 mg/kg; more preferably, the dosage of the anti-CD20 ADC is 0.5 mg/kg, 1 mg/kg, 1.5 mg/kg, 1.8 mg/kg, and 2.1 mg/kg; and most preferably, the dosage of the anti-CD20 ADC is 1.8 mg/kg.

16. The method according to claim 15, wherein the NHL patient is a R/R NHL patient after receiving at least two standard treatments, including FL patient, MZL patient, DLBCL patient, MCL patient, and SLL/CLL patient.

17. The method according to claim 16, wherein the NHL patient is a R/R DLBCL patient after receiving at least two standard treatments.

18. The method according to claim 17, wherein the DLBCL patient is a GCB type DLBCL patient and/or non-GCB type DLBCL patient.

19. The method according to claim 18, wherein the DLBCL patient is a non-GCB type DLBCL patient.

20. The method according to claim 19, wherein the dosage of the anti-CD20 ADC is 1.5 mg/kg or 1.8 mg/kg.

21. The method according to claim 17, wherein the DLBCL patient is a DLBCL patient with a baseline LDH of ≥ 250 and/or < 250.

22. The method according to claim 21, wherein the DLBCL patient is a DLBCL patient with a baseline LDH of ≥ 250.

23. The method according to claim 22, wherein the dosage of the anti-CD20 ADC is 1.8 mg/kg.

24. The method according to claim 17, wherein the DLBCL patient is a DLBCL patient with a baseline age of ≥ 60 and/or < 60.

25. The method according to claim 24, wherein the DLBCL patient is a DLBCL patient with a baseline age of ≥ 60.

26. The method according to claim 25, wherein the dosage of the anti-CD20 ADC is 1.8 mg/kg.

27. The method according to claim 17, wherein the DLBCL patient is a DLBCL patient with a baseline clinical phase of ≥ III and/or ≤ II.

28. The method according to claim 27, wherein the DLBCL patient is a DLBCL patient with a baseline clinical phase of ≥ III.

29. The method according to claim 28, wherein the dosage of the anti-CD20 ADC is 1.8 mg/kg.

30. The method according to any one of claims 14 to 29, wherein the anti-CD20 ADC can be administered once or multiple times; preferably, the anti-CD20 ADC is administered multiple times, and the administration frequency can be QW, Q2W, Q3W, or Q4W; and more preferably, the administration frequency is Q3W.

31. The method according to claim 30, wherein the anti-CD20 ADC is administered Q3W and continues to be administered until recovered, progressive disease (PD), or death.
